# EUROPÄISCHE PATENTSCHRIFT

(11) **EP 2 704 634 B1**
(45) Veröffentlichungstag und Bekanntmachung des Hinweises auf die Patenterteilung: **14.02.2018**
(21) Anmeldenummer: 12709509.9
(22) Anmeldetag: 05.03.2012
(51) Int. Cl.: A61B 5/18, B60K 35/00, G01C 21/36

(54) **MULTIFUNKTIONSBEDIENEINRICHTUNG**
MULTIFUNCTION OPERATING DEVICE
DISPOSITIF DE COMMANDE MULTIFONCTION

(30) Priorität: 12.03.2011 DE 102011013757; 03.09.2011 DE 102011112445
(43) Veröffentlichungstag der Anmeldung: 12.03.2014
(73) Patentinhaber: Volkswagen Aktiengesellschaft, 38436 Wolfsburg (DE)
(72) Erfinder: BECKMANN, Mark, 38124 Braunschweig (DE)
(86) Internationale Anmeldenummer: PCT/EP2012/000973
(87) Internationale Veröffentlichungsnummer: WO 2012/123075

(56) Entgegenhaltungen:
- EP-A2- 1 870 673
- WO-A1-2009/143903
- DE-A1- 10 228 703
- DE-A1-102006 050 017
- US-A1- 2010 293 462

## Beschreibung

Die vorliegende Anmeldung betrifft eine Multifunktionsbedieneinrichtung sowie ein Verfahren zum Betreiben einer derartigen Multifunktionsbedieneinrichtung in Fahrzeugen, beispielsweise in Kraftfahrzeugen, und entsprechend ausgerüstete Fahrzeuge.

Multifunktionselemente und Multifunktionsbedieneinrichtungen für Kraftfahrzeuge sind in vielfältiger Art bekannt. Mit derartigen Multifunktionsbedieneinrichtungen können verschiedene Geräte in einem Kraftfahrzeug mit einer einzigen Bedieneinrichtung gesteuert werden, beispielsweise Radio, CD-Spieler, Navigationssystem, Bordcomputer oder Telefonanlage. Multifunktionsbedieneinrichtungen sind beispielsweise aus der EP 0 701 926 A2 bekannt. In dieser Druckschrift wird eine Multifunktionsbedieneinrichtung offenbart, bei welcher ein Bildschirm sowie ein oder mehrere Tastenfelder Verwendung finden.

Eine weitere Multifunktionsbedieneinrichtung ist aus der DE 199 41 963 A1 bekannt, bei welcher ebenso ein Bildschirm sowie randseitig am Bildschirm angeordnete Bedientasten verwendet werden.

Eine weitere Möglichkeit zur Bedienung, welche bei elektronischen Geräten zunehmend eingesetzt wird, ist die Bedienung mittels berührungsempfindlicher Bildschirme, sogenannter Touchscreens. Hier können Bedienelemente wie Taster oder Schieberegler direkt auf dem berührungsempfindlichen Bildschirm dargestellt werden und durch eine Berührung dieses Bildschirms bedient werden. Derartige Touchscreens kommen beispielsweise verbreitet bei Mobiltelefonen zum Einsatz. Auf diese Weise können flexibel verschiedene Bedienoberflächen zur Bedienung verschiedener Funktionen bzw. Vorrichtungen auf dem Bildschirm dargestellt werden.

Bei einer Verwendung einer Multifunktionsbedieneinrichtung mit einem berührungsempfindlichen Bildschirm zur Darstellung verschiedener Bedienoberflächen kann jedoch das Problem auftreten, dass für manche Anwendungen die Bedienoberfläche so komplex ist, dass die Bedienung beispielsweise einen Fahrer eines Kraftfahrzeuges so sehr ablenken könnte, dass ein sicheres Fahren des Fahrzeuges nicht mehr gewährleistet wäre.

Eine derartige Ablenkung des Fahrers kann insbesondere dann geschehen, wenn die Multifunktionsbedieneinrichtung als Client dient und die Anwendung auf einem Server läuft, wobei der Server in manchen Fällen auch ein Mobiltelefon sein kann, sodass in einem derartigen Fall eine Anwendung des Mobiltelefons auf der Multifunktionsbedieneinrichtung dargestellt wird. Derartige Anwendungen auf Mobiltelefonen sind im Regelfall nicht für die Nutzung in einem Fahrzeug optimiert und können daher besonders leicht zu einer Ablenkung führen.
Aus der DE 100 39 795 C2 ist es diesbezüglich bekannt, allgemein eine Aufmerksamkeit eines Fahrers zu überwachen und ggf. eine Warnung auszugeben. Aus der US 6,262,657 B1 ist es bekannt, einen Fahrer zu warnen, wenn ein Telefonieren mit einem Mobiltelefon zu einer instabilen Fahrsituation führt.

Aus EP 1 870 673 A2, DE 10 2006 050017 A1, DE 102 28 703 A1 und WO 2009/143903 A1 ist bekannt, den Detailierungsgrad der Darstellungen in Navigationssystemen an schwierige Verkehrssituationen anzupassen. Es ist daher eine Aufgabe der vorliegenden Erfindung, Bedieneinrichtungen und entsprechende Verfahren bereitzustellen, bei welchen das Risiko einer Ablenkung eines Fahrers in einem Kraftfahrzeug reduziert ist.

Diese Aufgabe wird gelöst durch ein Verfahren nach Anspruch 1 und eine Vorrichtung nach Anspruch 20. Ein erfindungsgemäßes Verfahren umfasst ein Bereitstellen von Daten zur Darstellung einer Anwendung auf einem Bildschirm, ein Bereitstellen von Informationen hinsichtlich eines Ablenkungsgrades und ein Darstellen der Anwendung auf dem Bildschirm in Abhängigkeit von den Informationen bezüglich des Ablenkungsgrades und einer Fahrsituation eines Fahrzeugs.
Das Darstellen der Anwendung in Abhängigkeit von den Informationen hinsichtlich des Ablenkungsgrades und einer Fahrsituation des Fahrzeugs kann ein Entscheiden, ob die Anwendung auf dem Bildschirm dargestellt wird, umfassen.

Das Bereitstellen von Informationen hinsichtlich eines Ablenkungsgrades umfasst erfindungsgemäß ein Bereitstellen von Informationen hinsichtlich eines Ablenkungsgrades der Anwendung, d.h. das Bereitstellen von Informationen, welche charakteristisch dafür sind, wie sehr die Anwendung dafür geeignet ist, einen Fahrer oder anderen Bediener der Anwendung abzulenken.
Auf diese Weise kann flexibel entschieden werden, ob und wie in einer bestimmten Fahrsituation die Anwendung auf dem Bildschirm dargestellt werden kann, ohne die Fahrsicherheit in inakzeptabler Weise zu beeinträchtigen.
Das Bereitstellen von Informationen hinsichtlich des Ablenkungsgrades kann insbesondere ein Bereitstellen von Informationen bezüglich darzustellender Benutzerschnittstellenelemente der Anwendung auf dem Bildschirm umfassen. Dabei bedeuten beispielsweise eine Vielzahl von Benutzerschnittstellenelementen, kleine Benutzerschnittstellenelemente und/oder kompliziert zu bedienende Benutzerschnittstellenelemente einen höheren Ablenkungsgrad.

Das Verfahren kann dabei zudem ein Berechnen einer Größe der Benutzerschnittstellenelemente auf dem Bildschirm umfassen, da eine Anwendung ggf. auf verschiedenen Bildschirmgrößen dargestellt werden kann und somit der Ablenkungsgrad auch von der Bildschirmgröße abhängen kann.

Zudem kann das Verfahren ein Bestimmen einer maximalen Anzahl von Benutzerschnittstellenelementen umfassen. Beispielsweise können die Informationen bezüglich Benutzerschnittstellenelementen ergeben, dass in manchen Fällen weniger Benutzerschnittstellenelemente und in anderen Fällen mehr Benutzerschnittstellenelemente dargestellt werden. Für das Entscheiden kann dann beispielsweise die maximale Anzahl von Benutzerschnittstellenelementen verwendet werden.

Zusätzlich oder alternativ zu Informationen hinsichtlich des Ablenkungsgrades der Anwendung kann das Bereitstellen von Informationen hinsichtlich eines Ablenkungsgrades auch ein Erfassen von Informationen bezüglich eines Ablenkungsgrades eines Fahrers des Fahrzeugs auf Basis von Sensorinformationen, beispielsweise auf Basis von Bildern einer Kamera oder auf Basis von Informationen von Bedienelementen des Fahrzeugs, umfassen. Durch Auswerten der Sensorinformationen können beispielsweise Bewegungsmuster des Fahrers erfasst werden, welche auf eine Ablenkung hindeuten.

Das Bereitstellen von Daten zur Darstellung einer Anwendung und/oder das Bereitstellen von Informationen hinsichtlich des Ablenkungsgrades kann ein Senden der Daten von einem Server zu einem Client, wobei der Client den Bildschirm umfasst, umfassen. Das Entscheiden kann dann beispielsweise im Client durchgeführt werden. Es ist jedoch auch ein serverseitiges Entscheiden möglich.

Das Bereitstellen von Informationen hinsichtlich des Ablenkungsgrades und das Darstellen der Anwendung in Abhängigkeit von den Informationen wird bei manchen Ausführungsbeispielen nur dann durchgeführt, wenn die Anwendung eine fahrzeugfremde Anwendung, d.h. eine nicht fest im Fahrzeug vorgesehene Anwendung, ist. Derartige fahrzeugfremde Anwendungen können beispielsweise von einem fahrzeugfremden Server, beispielsweise einem Mobiltelefon, zu dem den Bildschirm umfassenden Client übertragen werden.

Das Senden der Daten zur Darstellung der Anwendung auf dem Bildschirm kann dabei insbesondere nach dem Terminal Mode Standard erfolgen.

Das Darstellen erfolgt erfindungsgemäß zusätzlich in Abhängigkeit von einer Position des Bildschirms in einem Fahrzeug. Beispielsweise sind Bildschirme, auf welche ein Fahrer Zugriff hat, kritischer als Bildschirme, welche beispielsweise nur Fondinsassen zur Verfügung stehen.
Die Fahrsituation kann insbesondere ein Fahren des Fahrzeugs oder ein Stehen des Fahrzeugs und/oder ein Zustand einer Zündung des Fahrzeugs sein. Auch die Geschwindigkeit des Fahrzeugs oder ein Einsatz von Fahrassistenten kann berücksichtigt werden.
Entsprechende Vorrichtungen, insbesondere entsprechende Clients und entsprechende Server, werden ebenfalls bereitgestellt.
Die Erfindung wird nachfolgend unter Bezugnahme auf die beigefügte Zeichnung anhand von Ausführungsbeispiel näher erläutert. Es zeigen:
- Figur 1: ein Fahrzeug mit einem Ausführungsbeispiel eines erfindungsgemäßen Systems,
- Figur 2: ein Flussdiagramm zur Veranschaulichung eines Ausführungsbeispiels eines erfindungsgemäßen Verfahrens, und
- Figur 3: ein Flussdiagramm zur Veranschaulichung eines weiteren Ausführungsbeispiels eines erfindungsgemäßen Verfahrens.
Im Folgenden werden Ausführungsbeispiele der Erfindung unter Bezugnahme auf die beigefügte Zeichnung detailliert erläutert. Es ist zu bemerken, dass diese Ausführungsbeispiele lediglich als Beispiel dienen und nicht als einschränkend auszulegen sind. Insbesondere ist die Beschreibung eines Ausführungsbeispiels mit einer Vielzahl von Merkmalen nicht dahingehend auszulegen, dass alle diese Merkmale zur Ausführung der Erfindung nötig sind, da andere Ausführungsbeispiele weniger Merkmale und/oder alternative Merkmale aufweisen können. Zudem ist zu bemerken, dass Merkmale verschiedener Ausführungsbeispiele miteinander kombiniert werden können, sofern nichts anderes angegeben ist.

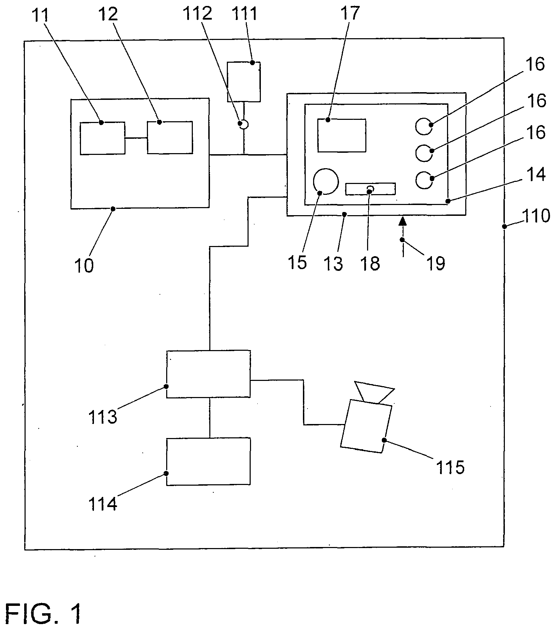

In Fig. 1 ist ein Fahrzeug 110, beispielsweise ein Kraftfahrzeug, dargestellt, welches ein erfindungsgemäßes System 10 bis 19 umfasst. Das erfindungsgemäße System umfasst bei dem Ausführungsbeispiel der Figur 1 ein Steuergerät 10, welches wie nachfolgend näher erläutert werden wird als Server arbeitet, und eine Multifunktionsbedieneinrichtung 13, welche als Client arbeitet.

Das Steuergerät 10 kann zur Steuerung einer oder mehrerer Funktionen in dem Fahrzeug 110 eingerichtet sein. Beispiele für derartige Funktionen sind beispielsweise eine Radiofunktion, ein Bordcomputer, eine Telefonfunktion, ein Navigationssystem, ein CD-Spieler oder eine Heizung des Kraftfahrzeugs. Bei dem dargestellten Ausführungsbeispiel umfasst das Steuergerät 10 einen Prozessor 11 und einen Speicher 12, wobei in dem Speicher 12 Programme abgelegt werden können, die bei Ausführung auf dem Prozessor 11 die Steuerfunktionen des Steuergeräts 10 implementieren.

Die Multifunktionsbedieneinrichtung 13 umfasst einen Bildschirm 14, welcher bei dem dargestellten Ausführungsbeispiel ein berührungsempfindlicher Bildschirm, ein so genannter Touchscreen, ist.

Zur Bedienung von Steuerfunktionen des Steuergeräts 10 durch einen Benutzer, beispielsweise einen Fahrer des Fahrzeugs 110, werden von dem Steuergerät 10 Daten zur Darstellung einer entsprechenden Anwendung, insbesondere einer Bedienoberfläche für die Anwendung, auf dem Bildschirm 14 gesendet. In Figur 1 sind dabei als Beispiel ein Drehregler 15, welcher beispielsweise durch eine kreisförmige Bewegung eines Fingers auf dem Bildschirm bedient werden kann, verschiedene Knöpfe 16, ein Anzeigebereich 17 zur Anzeige von Daten und ein Schieberegler 18 auf dem Bildschirm 14 dargestellt. Mit diesen Bedienelementen können durch einen Benutzer ein oder mehrere der Steuerfunktionen des Steuergeräts 10 bedient werden. Dabei ist sowohl eine kombinierte Darstellung mehrerer Steuerfunktionen, d.h. Bedienelemente für verschiedene Steuerfunktionen bei einer einzigen Anzeige, als auch ein Wechsel der Darstellung zwischen verschiedenen Steuerfunktionen möglich. Neben den auf dem Bildschirm 14 dargestellten Benutzerschnittstellenelementen können in der Multifunktionsbedieneinrichtung 13 auch weitere "Hardware"-Benutzerschnittstellenelemente wie Knöpfe oder Drehregler bereitgestellt sein.

Die Übertragung zwischen dem als Server dienenden Steuergerät 10 und der als Terminal dienenden Multifunktionsbedieneinrichtung 13 kann beispielsweise nach dem Terminal Mode Standard erfolgen. Die Übertragung kann auch über eine Video- und/oder eine Datenschnittstelle erfolgen.

Zusätzlich oder alternativ zu den Daten, welche die Multifunktionsbedieneinrichtung 14 von dem Steuergerät 10 empfängt, kann die Multifunktionsbedieneinrichtung 13 auch Daten zur Darstellung einer Anwendung von einem mobilen Gerät 111, beispielsweise einem Mobiltelefon, empfangen, welches über eine Schnittstelle 112 mit der Multifunktionsbedieneinrichtung 13 koppelbar ist. Insbesondere moderne Mobiltelefone, so genannte Smartphones, können eine Vielzahl von Anwendungen darstellen, welche beispielsweise auch Spiele oder Videos sein können. Auch die Daten von dem mobilen Gerät 111 können beispielsweise nach dem Terminal Mode Standard an die Multifunktionsbedieneinrichtung 13 übertragen werden.

Bei anderen Ausführungsbeispielen kann das mobile Gerät 111 auch beispielsweise nach dem Terminal Mode Standard Daten zur Darstellung auf der Multifunktionsbedieneinrichtung 14 an das Steuergerät 10 übertragen, und das Steuergerät 10 kann dann in Abhängigkeit von diesen Daten die Multifunktionsbedieneinrichtung 13 zur Darstellung einer entsprechenden Anwendung ansteuern, beispielsweise unter Benutzung der bereits erwähnten Video- und/oder Datenschnittstelle.

Zusammen mit den Daten zur Darstellung der Anwendung übertragen bei dem Ausführungsbeispiel der Figur 1 das Steuergerät 10 und/oder das mobile Gerät 111 Informationen, welche einen Ablenkungsgrad der Anwendung kennzeichnen, an die Multifunktionsbedieneinrichtung 13. In Abhängigkeit von dieser Information und einer Fahrsituation, welche die Multifunktionsbedieneinrichtung wie durch einen Pfeil 19 gekennzeichnet erhält, entscheidet die Multifunktionsbedieneinrichtung 13 über die Darstellung der Anwendung, z.B. ob die Anwendung, in diesem Fall die Bedienoberfläche, auf dem Bildschirm dargestellt wird. Die Fahrsituation kann beispielsweise beschreiben, ob das Fahrzeug steht oder ob es sich bewegt, beispielsweise durch Erhalten eines Signals von einem Geschwindigkeitsmesser des Fahrzeugs. Ein entsprechendes Signal kann bei einem Ausführungsbeispiel auch über das Steuergerät 10 geliefert werden. Beispielsweise kann bei einem fahrenden Fahrzeug eine Bedienoberfläche, deren Ablenkungsgrad einen vorgegebenen Schwellenwert überschreitet, nicht mehr dargestellt werden, während eine derartige Bedienoberfläche bzw. Anwendung bei einem stehenden Fahrzeug angezeigt wird. Ein weiteres Beispiel für eine Fahrsituation ist Zündung ein oder aus, sodass festgestellt werden kann, dass das Fahrzeug nicht nur steht, sondern auch ausgeschaltet ist.

Die Informationen hinsichtlich des Ablenkungsgrades können insbesondere Informationen über die auf dem Bildschirm darzustellenden Benutzerschnittstellenelemente wie beispielsweise die Elemente 15 bis 18, d.h. Bedienelemente und Informationswiedergabeelemente, umfassen. Derartige Benutzerschnittstellenelemente werden auch als GUI-Elemente (General User Interface) bezeichnet. Insbesondere kann in den Informationen hinsichtlich des Ablenkungsgrades angegeben werden, welche Benutzerschnittstellenelemente darzustellen sind und welche Größe sie haben. Hierzu kann das Steuergerät 10 und/oder die Multifunktionsbedieneinrichtung 13 in Abhängigkeit von einer Größe des Bildschirms 14 die tatsächliche dargestellte Größe der Benutzerschnittstellenelemente bestimmen. Beispielsweise deuten viele Benutzerschnittstellenelemente und/oder kleine Benutzerschnittstellenelemente auf einen hohen Ablenkungsgrad hin, während wenige Benutzerschnittstellenelemente und/oder große Benutzerschnittstellenelemente auf einen niedrigen Ablenkungsgrad hindeuten.

Somit kann beispielsweise die Anzahl der Benutzerschnittstellenelemente oder die Größe der Benutzerschnittstellenelemente mit einem jeweiligen vorgegebenen Schwellenwert verglichen werden, wobei dieser Schwellenwert von der Fahrsituation abhängen kann.

Zu bemerken ist, dass die obigen Schwellenwerte bzw. die Entscheidung, ob die Darstellung erfolgt, auch von der Platzierung der Multifunktionsbedieneinrichtung 13 in dem Fahrzeug 110 abhängen kann. Beispielsweise ist eine Multifunktionsbedieneinrichtung, welche derart angeordnet ist, dass sie nur von einem Beifahrer oder sogar nur von Fondinsassen eines Kraftfahrzeugs bedient werden kann, unkritischer als eine Multifunktionsbedieneinrichtung, welche zur Bedienung durch einen Fahrer angeordnet ist.

Weiter ist zu bemerken, dass bei manchen Ausführungsbeispielen das Senden von Informationen, welche einen Ablenkungsgrad der Anwendung kennzeichnen, und die entsprechende Auswertung dieser Informationen nur für das mobile Gerät 111, nicht jedoch für das Steuergerät 10 durchgeführt wird. Bei derartigen Ausführungsbeispielen wird davon ausgegangen, dass durch das Steuergerät 10, welches fest im Fahrzeug verbaut ist, nur fahrzeugeigene Anwendungen zur Darstellung gelangen, welche insbesondere für die Verwendung in einem Fahrzeug konzipiert sind und daher im Allgemeinen einen relativ geringen Ablenkungsgrad aufweisen. Bei Anwendungen, welche vom mobilen Gerät 111 kommen, handelt es sich demgegenüber um fahrzeugfremde Anwendungen, über welche z.B. der Fahrzeughersteller keine Kontrolle hat und welche zumindest in vielen Fällen nicht für die Verwendung in einem Fahrzeug, insbesondere in einem fahrenden Fahrzeug, konzipiert sind und welche daher tendenziell einen höheren Ablenkungsgrad aufweisen. Es ist jedoch selbstverständlich auch möglich, die oben beschriebene Vorgehensweise sowohl für das Steuergerät 10 als auch für das mobile Gerät 111 durchzuführen.

Zudem ist zu bemerken, dass bei manchen Ausführungsbeispielen nicht alle auf der Multifunktionsbedieneinrichtung 13 laufenden Anwendungen von einem Server wie dem Steuergerät 10 oder dem mobilen Gerät 111 kommen müssen, sondern auch auf der Multifunktionsbedieneinrichtung fest installiert sein können. Mit derartigen fest installierten Anwendungen können beispielsweise Grundfunktionen des Fahrzeugs bedienbar sein, wie Radio oder Klimaanlage. Bei derartigen Anwendungen wird bei manchen Ausführungsbeispielen auch keine Überprüfung durchgeführt. Selbstverständlich können Anwendungen zur Bedienung derartiger grundlegender Fahrzeugfunktionen ebenso von dem Steuergerät 10 übertragen werden.

Zu bemerken ist auch, dass bei manchen Ausführungsbeispielen, wenn der Ablenkungsgrad der Anwendung einen vorgegebenen Schwellenwert überschreitet, bei einem Bediener nachgefragt werden kann, ob die Anwendung dargestellt werden soll. Der Anwender, beispielsweise der Fahrer des Fahrzeugs, kann sich dann gleichsam entscheiden, die Anwendung auf eigenes Risiko laufen zu lassen. Es ist jedoch zu bemerken, dass dies optional ist, und bei anderen Ausführungsbeispielen aus Sicherheitsgründen eine Anwendung, deren Ablenkungsgrad einen z.B. in Abhängigkeit von einer Fahrsituation vorgegebenen Schwellenwert überschreitet, grundsätzlich nicht dargestellt wird.

Wenn eine Anwendung auf Basis ihrer Ablenkungsgrades nicht dargestellt wird, kann bei manchen Anwendungen eine alternative Anwendung, beispielsweise eine fahrzeugeigene Anwendung wie eine Radioanwendung, dargestellt werden.

Im Folgenden werden einige Beispiele für Informationen über Benutzerschnittstellenelemente, welche eine Information hinsichtlich des Ablenkungsgrades der Anwendung darstellen können, erläutert.

Beispielsweise kann bei manchen Anwendungen ein so genanntes Menü auf dem Bildschirm dargestellt werden. Ein derartiges Menü kann beispielsweise im Anzeigebereich 17 dargestellt werden und/oder mehrere Knöpfe wie die Knöpfe 16 umfassen. Dabei kann die Information hinsichtlich der Benutzerschnittstellenelemente eine (maximale) Anzahl von Menüebenen, eine maximale Anzahl von sichtbaren Elementen und/oder eine maximale Anzahl von interaktiven Elementen wie Knöpfen oder Eingabefeldern umfassen, wobei die vorgenannten Informationen beispielsweise in Form von ganzzahligen Zahlenwerten übermittelt werden können. Je größer diese Zahlen, desto höher kann der Ablenkungsgrad eingestuft werden. Des Weiteren können Informationen übersendet werden, welche angeben, ob interaktive Elemente beispielsweise durch Farbe oder dreidimensionale Darstellung betont werden, ob bei interaktiven Elementen eine Audiorückkopplung erfolgt, d.h. eine akustische Rückmeldung, wenn das Element bedient wird, und/oder ob mit einem interaktiven Element bei verschiedenen Bedienweisen verschiedene Aktionen ausgelöst werden können, beispielsweise durch kurzes oder langes Drücken des gleichen Bedienknopfes. Diese Informationen können als Ja/Nein-Werte übermittelt werden. Ein Hervorheben interaktiver Elemente und eine akustische Rückkopplung erleichtern die Bedienung und verringern daher den Ablenkungsgrad, während interaktive Elemente, welche auf verschiedene Weise bedienbar sind, den Ablenkungsgrad erhöhen können.

Die Benutzerschnittstellenelemente können auch Text enthalten, wobei hinsichtlich des Textes Informationen übermittelt werden können. Beispielsweise kann übermittelt werden, ob animierter Text benutzt wird (beispielsweise in Form eines Ja/Nein-Wertes), beispielsweise blinkende Buchstaben, die Anzahl verschiedener Textgrößen in einem Menü und/oder auf einem Bildschirm kann übermittelt werden, beispielsweise als ganzzahliger Wert, es kann übermittelt werden, ob hervorgehobener Text verwendet wird (beispielsweise als Ja/Nein-Wert), es kann übermittelt werden, ob laufender Text, d.h. sich durch ein Fenster bewegender Text, verwendet wird (als Ja/Nein-Wert), und in diesem Fall kann optional zusätzlich beispielsweise als ganzzahliger Wert angegeben werden, wie lange es dauert, bis der Text vollständig durchgelaufen ist. Die Verwendung von animiertem Text, von verschiedenen Textgrößen in einem Menü, die Verwendung von laufendem Text, insbesondere wenn es länger dauert, bis der Text durchgelaufen ist, kann den Ablenkungsgrad erhöhen. Die Verwendung von hervorgehobenem Text kann zumindest teilweise den Ablenkungsgrad erniedrigen, da es dann einfacher ist, wichtige Stellen zu erkennen.

Des Weiteren kann in Form eines Ja/Nein-Wertes angegeben werden, ob eine Texteingabe benötigt ist, was den Ablenkungsgrad erhöhen kann. Ist dies der Fall, kann beispielsweise angegeben werden, ob Hilfen zur Texteingabe, beispielsweise ein Buchstabierprogramm oder eine Spracheingabe, welche den Ablenkungsgrad erniedrigen können, vorhanden sind. Schließlich kann die Gesamtzahl von dargestellten Buchstaben bzw. die maximale Anzahl von Buchstaben, welche für eine Anwendung auf dem Bildschirm dargestellt ist, angegeben werden, wobei eine höhere Anzahl von Buchstaben einem höheren Ablenkungsgrad zugeordnet werden kann, da es beispielsweise länger dauern kann, den gesamten Text zu lesen.

Hinsichtlich der in derartigen Texten verwendeten Buchstaben kann eine minimale Höhe und/oder eine minimale Breite, jeweilig als ganzzahliger Wert, angegeben werden, wobei die Höhe und Breite wie oben bereits erwähnt beispielsweise in Abhängigkeit von der Größe und/oder Auflösung des Bildschirms beispielsweise durch den jeweiligen Server, im Falle von Figur 1 durch das Steuergerät 10, berechnet werden kann. Größere Werte bedeuten im Allgemeinen eine leichtere Lesbarkeit und somit einen geringeren Ablenkungsgrad. Zudem kann beispielsweise in Form eines Ja/Nein-Wertes übermittelt werden, ob optimierte Schriftgrößen verwendet werden können, beispielsweise durch Verwendung skalierbarer Zeichensätze, was die Lesbarkeit erleichtern kann und somit den Ablenkungsgrad verringern kann.

Ebenso können bei manchen Ausführungsbeispielen auf dem Bildschirm der Multifunktionsbedieneinrichtung Bilder dargestellt werden. In diesem Fall kann als Information die maximale Anzahl dargestellter Bilder, die minimale Höhe beispielsweise des kleinsten Bildes und die minimale Breite beispielsweise des kleinsten Bildes übermittelt werden, wobei diese Informationen jeweils als ganzzahlige Werte übermittelt werden können. Höhe und Breite können wie oben für die Buchstabengrößen erläutert in Abhängigkeit von einer Bildschirmgröße und Bildschirmauflösung ermittelt werden. Eine höhere Anzahl von Bildern bzw. kleinere Bilder können einem höheren Ablenkungsgrad zugeordnet werden.

Die Informationen über Benutzerschnittstellenelemente können auch Informationen über anzuzeigende Listen umfassen. Derartige Listen können beispielsweise verwendet werden, wenn ein Benutzer aus mehreren Elementen ein oder mehrere Elemente auswählt. Insbesondere können verschiedene Ja/Nein-Informationen übermittelt werden, welche das Vorhandensein verschiedener Möglichkeiten der Bedienung derartiger Listen kennzeichnen.

Beispielsweise kann angegeben werden, ob eine Liste durch langes Drücken des Bildschirms an geeigneten Stellen durchgeblättert ("gescrollt") werden kann, ob durch das lange Drücken und/oder durch ein Entlanggleiten mit dem Finger eine Veränderung einer Scrollgeschwindigkeit möglich ist oder ob eine so genannte Scrollbar zur Verfügung steht. Derartige Elemente können die Bedienung vereinfachen. Des Weiteren kann angegeben werden, ob ein Schwenken der Liste verfügbar ist, beispielsweise mittels Pfeilen, ob die Verwendung eines Cursors unterstützt ist, d.h. einer Markierung zur Eingabe von Text, ob in der Liste Knöpfe verwendet werden und/oder ob ein Scrollen der Liste nötig ist, um derartige Knöpfe zu erreichen. Die Verwendung von Knöpfen und auch das Scrollen zum Erreichen derartiger Knöpfe macht die Bedienung einer Liste im Regelfall schwieriger und kann daher den Ablenkungsgrad erhöhen.

Auch Informationen hinsichtlich verwendeter Knöpfe ("Buttons") können als Beispiel für Information hinsichtlich Benutzerschnittstellenelemente übermittelt werden. Zu derartigen Knöpfen können beispielsweise auch Links beispielsweise beim Anzeigen von Internetseiten gerechnet werden, da bei Drücken auf einen derartigen Link eine Aktion, beispielsweise das Anzeigen einer entsprechenden neuen Seite, ausgelöst wird.

Beispielsweise kann eine minimale Höhe und eine minimale Breite von verwendeten Knöpfen gesendet werden, wobei die minimale Höhe und minimale Breite wie oben bereits für Zeichen oder Bilder erläutert in Abhängigkeit von einer Bildschirmgröße und/oder -auflösung berechnet werden kann. Größere Knöpfe vereinfachen die Bedienung und entsprechen dementsprechend einem niedrigeren Ablenkungsgrad.

Des Weiteren kann als ganzzahliger Wert eine Entfernung zwischen zwei interaktiven Gebieten auf dem Bildschirm übergeben werden, wobei diese Distanz wiederum in Abhängigkeit von der Bildschirmgröße berechnet werden kann. Des Weiteren kann angegeben werden, nach welchem Prinzip Knöpfe arbeiten, beispielsweise ob durch Drücken des Knopfes eine Aktion ausgelöst wird, durch Loslassen eines Knopfes eine Aktion ausgelöst wird oder beides. Schließlich kann die Anzahl von Umschaltknöpfen auf einem Bildschirm einer Anwendung als ganzzahliger Wert übergeben werden, wobei eine höhere Anzahl von Knöpfen im Allgemeinen die Bedienung erschweren kann und somit einem größeren Ablenkungsgrad zugeordnet ist.

Des Weiteren kann als Ja/Nein-Wert übergeben werden, ob Schieberegler wie der Schieberegler 18 der Figur 1 verwendet werden. In gleicher Weise kann als Ja/Nein-Wert übergeben werden, ob Fortschrittsbalken im Falle von lang andauernden Aktivitäten verwendet werden, wobei ein derartiger Fortschrittsbalken eine Ablenkung darstellen kann. Auch die Verwendung von Piktogrammen, so genannter Icons, kann mittels einer oder mehrerer Variablen charakterisiert werden. Beispielsweise kann die Anzahl derartiger Piktogramme, die minimale Höhe von verwendeten Piktogrammen oder die minimale Breite von verwendeten Piktogrammen jeweils als ganzzahliger Wert übergeben werden, wobei die minimale Höhe bzw. die minimale Breite jeweils als ganzzahliger Wert übergeben werden können: Höhe und Breite können wiederum wie bereits oben erläutert in Abhängigkeit von der Bildschirmgröße und/oder -auflösung berechnet werden.

Des Weiteren kann in Form eines Ja/Nein-Wertes übergeben werden, ob eine jeweilige Anwendung eine spezielle Betriebsart für Fahrzeuge unterstützt, beispielsweise eine Betriebsart, in welcher die Bedienung vereinfacht ist.

Eine Zeit, beispielsweise in Millisekunden, zwischen dem Beginn einer Interaktion und dem Erhalten von einer visuellen oder akustischen Rückmeldung kann ebenso übergeben werden, wobei ein höherer Wert den Ablenkungsgrad erhöhen kann. Des Weiteren kann in Form eines Ja/Nein-Wertes übergeben werden, ob eine bestimmte Anwendung Inhalte aus dem Internet, insbesondere aus dem WWW (World Wide Web) benutzt, welche insbesondere außerhalb der Kontrolle der Anwendung sind. Die Benutzung derartiger Elemente kann den Ablenkungsgrad erhöhen.

Schließlich kann in Form eines Ja/Nein-Wertes angegeben werden, ob Nichtstandardbenutzerschnittstellenelemente, beispielsweise so genannte Soft-DDS, verwendet werden. Die Verwendung derartiger nicht standardisierter Elemente kann ebenfalls den Ablenkungsgrad erhöhen.

Die obigen Beispiele für Informationen hinsichtlich Benutzerschnittstellenelemente können einzeln oder auch zusammen in verschiedenen Ausführungsbeispielen verwendet werden. Bei anderen Ausführungsbeispielen kann, beispielsweise auf Basis der verwendeten Benutzerschnittstellenelemente, z.B. durch einen Hersteller einer Anwendung ein einziger Wert, welcher den Ablenkungsgrad kennzeichnet, bereitgestellt werden und verwendet werden.

Bei anderen Ausführungsbeispielen kann eine Information hinsichtlich eines Ablenkungsgrades bereits eine Information sein, ob eine Anwendung in einer bestimmten Fahrsituation angezeigt werden kann. Eine derartige Information kann beispielsweise eine Zertifizierung einer jeweiligen Anwendung umfassen, wobei die Zertifizierung durch eine Zertifizierungsstelle erfolgt, welche beispielsweise mit einem entsprechenden Zertifikat bestätigt, dass die Anwendung in einer bestimmten Fahrsituation dargestellt werden darf. Bei einem beabsichtigten Start der Anwendung, beispielsweise durch eine Benutzereingabe, kann dann bei manchen Ausführungsbeispielen das Zertifikat an die Multifunktionsbedieneinrichtung 14 und/oder das Steuergerät 10 (wenn die Anwendung auf dem mobilen Gerät 111 läuft) gesendet werden. Bei anderen Ausführungsbeispielen kann beispielsweise bei einer Anwendung das mobile Gerät 111 selbst überprüfen, ob die Anwendung für eine gegebene Fahrsituation zertifiziert ist, und dann der Multifunktionsbedieneinrichtung 14 und/oder dem Steuergerät 10 lediglich mitteilen, ob die Anwendung gestartet werden kann. Eine derartige Zertifizierung kann auch fahrzeugabhängig erfolgen, sodass das Ausführen der Anwendung nicht nur in Abhängigkeit von der Fahrsituation, sondern auch in Abhängigkeit von dem jeweiligen Fahrzeugtyp erfolgen kann. Eine derartige Information hinsichtlich der Kompatibilität der Anwendung mit bestimmten Fahrzeugtypen kann jedoch auch unabhängig von einer Zertifizierung vorgesehen sein.

Zu bemerken ist weiterhin, dass das Ausführungsbeispiel der Figur 1 nur als Beispiel zu verstehen ist und verschiedene Variationen möglich sind. Beispielsweise kann mehr als ein Steuergerät bzw. können verschiedene Server vorhanden sein, welche Daten hinsichtlich verschiedener Anwendungen zu der Multifunktionsbedieneinrichtung 13 oder zu dem Steuergerät 10 übertragen. Auf der anderen Seite können in dem Fahrzeug 110 auch mehrere Multifunktionsbedieneinrichtungen 13 vorhanden sein, beispielsweise für verschiedene Insassen des Fahrzeuges. Dabei kann es, wie bereits erläutert, von der Positionierung der Multifunktionsbedieneinrichtung abhängen, ob bei einer bestimmten Fahrsituation eine bestimmte Anwendung dargestellt wird.

Während bei dem Ausführungsbeispiel der Figur 1 die Entscheidung, ob eine Anwendung, insbesondere eine Benutzeroberfläche, dargestellt wird, in der Multifunktionsbedieneinrichtung 13 getroffen wird, kann bei anderen Ausführungsbeispielen diese Entscheidung auch in dem Steuergerät 10 getroffen werden, wobei hierzu die gleichen Informationen, d.h. eine Information hinsichtlich des Ablenkungsgrad und Daten über die Fahrsituation, herangezogen werden können.

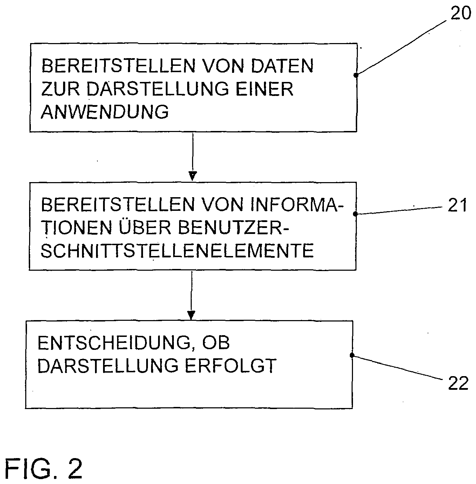

In Figur 2 ist ein Ausführungsbeispiel eines erfindungsgemäßen Verfahrens dargestellt, mit welchem beispielsweise die oben diskutierten Funktionen in der unter Bezugnahme auf Figur 1 beschriebenen Anwendung implementiert sein können.

In Schritt 20 werden Daten zur Darstellung einer Anwendung bereitgestellt, beispielsweise von dem Steuergerät 10 oder dem mobilen Gerät 111 für die Multifunktionsbedieneinrichtung 13.

In Schritt 21 werden Informationen über Benutzerschnittstellenelemente oder andere Informationen hinsichtlich eines Ablenkungsgrades der Anwendung bereitgestellt, beispielsweise ebenfalls von dem Steuergerät 10 der Figur 1 zu der Multifunktionsbedieneinrichtung 13 der Figur 1.

Bei Schritt 22 wird in Abhängigkeit von den Informationen von Schritt 21 und einer Fahrsituation entschieden, ob die Darstellung der Anwendung auf einem Bildschirm erfolgt.

Die unter Bezugnahme auf Figur 1 diskutierten Variationen und Abwandlungen sind ebenso auf das Verfahren der Figur 2 anwendbar.

Bei den oben diskutierten Ausführungsbeispielen werden Informationen über den Ablenkungsgrad einer Anwendung ausgewertet, um über die Darstellung der Anwendung, insbesondere darüber, ob die Anwendung dargestellt wird oder nicht, zu entscheiden. Bei dem Ausführungsbeispiel der Fig. 1 ist, wie im Folgenden näher erläutert werden wird, zudem die Möglichkeit vorgesehen, einen Ablenkungsgrad eines Fahrers eines Fahrzeugs auf Basis von Sensordaten zu erfassen und die Entscheidung über die Darstellung der Anwendung zusätzlich in Abhängigkeit von dem Ablenkungsgrad des Fahrers zu treffen.

Dabei ist zu bemerken, dass diese beiden Möglichkeiten, d.h. Entscheidung auf Basis von Informationen bezüglich des Ablenkungsgrades der Anwendung und Entscheidung auf Basis von Informationen über einen Ablenkungsgrad eines Fahrers nicht nur, wie im Falle des Ausführungsbeispiels der Fig. 1, zusammen implementiert sein können, sondern dass die zwei Möglichkeiten auch getrennt voneinander implementiert sein können. In anderen Worten wird bei manchen Ausführungsbeispielen nur eine Entscheidung auf Basis eines Ablenkungsgrades der Anwendung, bei manchen Ausführungsbeispielen nur eine Entscheidung auf Basis von Informationen über einen Ablenkungsgrad des Fahrers und bei manchen Ausführungsbeispielen beides verwendet.

Insbesondere weist die in dem Fahrzeug 110 der Fig. 1 installierte Vorrichtung eine Kamera 115 auf, mit welcher Bilder eines Fahrers des Fahrzeugs aufgenommen werden können. Die Bilder werden einer Auswerteeinrichtung 113 zugeführt. Es ist zu bemerken, dass die Auswerteeinrichtung 113 auch in die Multifunktionsbedieneinrichtung 13 oder in das Steuergerät 10 integriert sein kann, d.h. kein separates Element sein muss. Zusätzlich oder alternativ können der Auswerteeinrichtung 113 Signale von diversen Bedienelementen des Fahrzeugs 110 zugeführt werden, wobei entsprechende Sensoren der Bedienelemente durch ein Element 114 symbolisiert sind. Beispielsweise können Daten eines Lenkwinkelsensors oder Daten von Sensoren, welche beispielsweise Pedalstellungen von Gaspedal oder Bremspedal auswertet, durch die Sensoren 114 bereitgestellt werden.

Die Auswerteeinrichtung 113 wertet Signale der Kamera 115 und/oder der Sensoren 114 aus, um Informationen hinsichtlich eines Ablenkungsgrades eines Fahrers des Fahrzeugs 110 zu gewinnen.

Die Kamera 115 kann dabei eine Kamera, welche auf sichtbares Licht empfindlich ist, sein, kann aber auch eine Infrarotkamera sein. Die Kamera 115 kann beispielsweise auf einer Lenksäule des Fahrzeugs 110 oder an einem Himmel des Fahrzeugs 110 angebracht sein. Grundsätzlich ist jedoch jede Positionierung der Kamera 115 möglich, solange der Fahrer des Fahrzeugs 110 erfasst werden kann.

Die Auswerteeinrichtung 113 kann beispielsweise Bilder der Kamera auswerten, um zu überprüfen, ob und für welchen Zeitanteil ein Blick des Fahrers auf die Straße gerichtet ist. Auch kann analysiert werden, wie häufig der Fahrer den Blick weg von der Straße richtet. Je weniger der Blick zur Straße gerichtet ist, desto höher kann der Ablenkungsgrad des Fahrers eingestuft werden. Insbesondere kann auch festgestellt werden, ob der Blick des Fahrers und somit die Aufmerksamkeit des Fahrers auf den Bildschirm 14 gerichtet ist.

Zudem oder alternativ kann die Auswerteeinrichtung 113 Informationen der Sensoren 114 auswerten, um so ein Fahrverhalten des Fahrers zu analysieren, beispielsweise sein Lenkverhalten, ein Verhalten bei Bedienung der Pedale oder auch einen Geschwindigkeitsverlauf. Häufigere ruckartige Lenkradbewegungen können dabei beispielsweise auf einen hohen Ablenkungsgrad hindeuten.

Ähnlich wie obenstehend für die Informationen hinsichtlich des Ablenkungsgrades der Anwendung kann dann auch auf Basis der Informationen über den Ablenkungsgrad des Fahrers über die Darstellung der Anwendung entschieden werden und insbesondere die Anwendung nicht dargestellt werden bzw. eine Darstellung der Anwendung beendet werden, wenn der Ablenkungsgrad des Fahrers einen vorgegebenen Schwellenwert übersteigt. Optional kann auch eine Warnung, beispielsweise auf dem Bildschirm 14, ausgegeben werden, und/oder es kann, beispielsweise über den Bildschirm 14, abgefragt werden, ob die Anwendung weiter dargestellt werden soll, sodass der Fahrer sich dann entscheiden kann, die Anwendung auf eigene Verantwortung weiterlaufen zu lassen.

Ähnlich der Auswertung des Ablenkungsgrades der Anwendung kann die Vorrichtung der Fig. 1 derart eingerichtet sein, dass die Auswerteeinrichtung 113 nur aktiviert wird, wenn eine fahrzeugfremde Anwendung, beispielsweise eine durch das mobile Gerät 111 eingespeiste Anwendung, auf dem Bildschirm 14 angezeigt wird oder angezeigt werden soll und/oder wenn der Fahrer des Fahrzeugs mit der Multifunktionsbedieneinrichtung 13 interagiert. Bei Erkennen einer Ablenkung kann dann beispielsweise auf eine fahrzeugeigene Anwendung umgeschaltet werden.

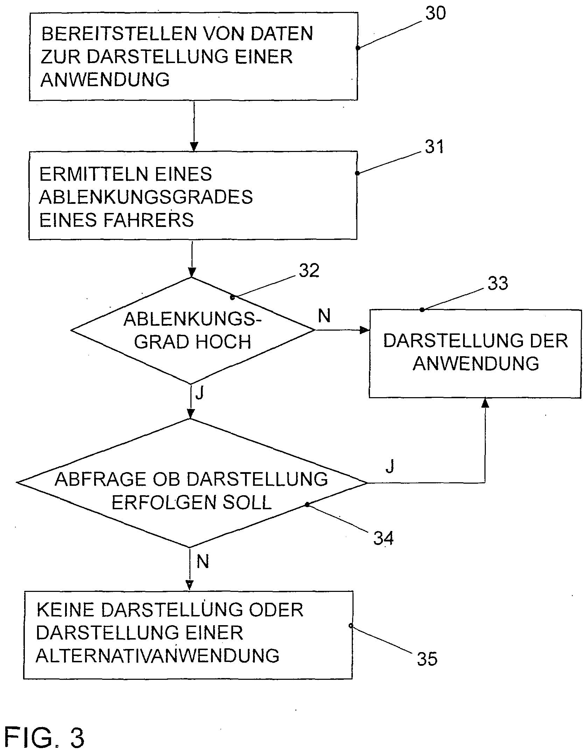

In Fig. 3 ist ein Ausführungsbeispiel eines erfindungsgemäßen Verfahrens veranschaulicht, welche ein Beispiel für die oben erläuterte Auswertung eines Ablenkungsgrades eines Fahrers darstellt und welches zum Beispiel in der Vorrichtung der Fig. 1 implementiert sein kann, jedoch auch unabhängig hiervon verwendbar ist.
In Schritt 30 werden Daten zur Darstellung einer Anwendung bereitgestellt, beispielsweise durch das Steuergerät 10 der Fig. 1 oder durch das mobile Gerät 11 der Fig. 1
In Schritt 31 wird ein Ablenkungsgrad eines Fahrers eines Fahrzeugs ermittelt, beispielsweise mittels der Auswerteeinrichtung 113 der Fig. 1.
In Schritt 32 wird überprüft, ob der Ablenkungsgrad des Fahrers hoch ist, z.B. einen vorgegebenen Schwellenwert überschreitet. Falls nein, wird bei Schritt 33 die Anwendung normal dargestellt bzw. mit der Darstellung der Anwendung fortgefahren. Falls der Ablenkungsgrad hoch ist, wird bei 34 abgefragt, ob die Darstellung der Anwendung erfolgen soll, d.h. der Fahrer des Fahrzeugs wird darum gebeten, die Darstellung zu bestätigen. Falls bestätigt wird, dass die Anwendung dargestellt werden soll, wird bei Schritt 33 wiederum die Anwendung dargestellt. Falls nein, wird in Schritt 35 die Anwendung nicht dargestellt und optional eine Alternativanwendung dargestellt.

## Patentansprüche

1. Verfahren zum Darstellen einer Anwendung in einem Fahrzeug (110), umfassend:
Bereitstellen von Daten zur Darstellung einer Anwendung auf einem Bildschirm (14),
Bereitstellen von Informationen hinsichtlich eines Ablenkungsgrades und
Darstellen der Anwendung auf dem Bildschirm (14) in Abhängigkeit von den Informationen hinsichtlich des Ablenkungsgrades und einer Fahrsituation des Fahrzeugs (110),
**dadurch gekennzeichnet, dass** das Bereitstellen von Informationen hinsichtlich eines Ablenkungsgrades ein Bereitstellen von Informationen hinsichtlich eines Ablenkungsgrades der Anwendung umfasst, und
dass das Darstellen in Abhängigkeit von einer Position des Bildschirms (14) in dem Fahrzeug (110) erfolgt.

2. Verfahren nach Anspruch 1, **dadurch gekennzeichnet, dass** das Darstellen der Anwendung in Abhängigkeit von den Informationen hinsichtlich des Ablenkungsgrades und der Fahrsituation des Fahrzeugs (110) ein Entscheiden, ob die Anwendung auf dem Bildschirm (14) dargestellt wird, umfasst.

3. Verfahren nach Anspruch 1 oder 2, **dadurch gekennzeichnet, dass** die Anwendung eine fahrzeugfremde Anwendung umfasst.

4. Verfahren nach Anspruch 3, **dadurch gekennzeichnet, dass** die Informationen hinsichtlich des Ablenkungsgrades Informationen über Benutzerschnittstellenelemente (15-18) der Anwendung umfassen.

5. Verfahren nach Anspruch 4, **dadurch gekennzeichnet, dass** das Verfahren weiterhin ein Berechnen einer Größe der Benutzerschnittstellenelemente (15-18) in Abhängigkeit von einer Größe und/oder Auflösung des Bildschirms (14) umfasst.

6. Verfahren nach Anspruch 4 oder 5, **dadurch gekennzeichnet, dass** das Darstellen in Abhängigkeit von einer Größe der Benutzerschnittstellenelemente, einer Anzahl der Benutzerschnittstellenelemente und/oder einer Ausgestaltung der Benutzerschnittstellenelemente erfolgt.

7. Verfahren nach einem der Ansprüche 1 bis 6, **dadurch gekennzeichnet, dass** das Bereitstellen von Informationen hinsichtlich eines Ablenkungsgrades ein Ermitteln von Informationen hinsichtlich eines Ablenkungsgrades eines Fahrers des Fahrzeugs auf Basis von Sensorinformationen umfasst.

8. Verfahren nach Anspruch 7, **dadurch gekennzeichnet, dass** die Sensorinformationen mindestens eine Information aus der Gruppe bestehend aus Bilddaten einer Kamera und Informationen von Bedienelementen des Fahrzeugs umfasst.

9. Verfahren nach Anspruch 7 oder 8, **dadurch gekennzeichnet, dass** das Erfassen des Ablenkungsgrades des Fahrers nur bei einer Interaktion des Fahrers mit einer den Bildschirm (14) umfassenden Bedieneinrichtung durchgeführt wird.

10. Verfahren nach einem der Ansprüche 1 bis 9, **dadurch gekennzeichnet, dass** die Information hinsichtlich eines Ablenkungsgrades ein Zertifikat der Anwendung oder eine von dem Zertifikat abgeleitete Information umfasst.

11. Verfahren nach einem der Ansprüche 1 bis 10, **dadurch gekennzeichnet, dass** die Fahrsituation ausgewählt ist aus der Gruppe, umfassend ein fahrendes Fahrzeug, ein stehendes Fahrzeug, ein Fahrzeug mit ausgeschalteter Zündung und ein Fahrzeug mit eingeschalteter Zündung.

12. Verfahren nach einem der Ansprüche 1 bis 11, **dadurch gekennzeichnet, dass** das Bereitstellen der Informationen hinsichtlich des Ablenkungsgrades ein Senden der Informationen von einem Server (10, 111) zu einem den Bildschirm (14) umfassenden Client umfasst.

13. Verfahren nach einem der Ansprüche 1 bis 12, **dadurch gekennzeichnet, dass** das Bereitstellen von Daten zur Darstellung einer Anwendung ein Übertragen der Daten von einem Server (10, 111) zu einem den Bildschirm (14) umfassenden Client (13) erfolgt.

14. Verfahren nach einem der Ansprüche 12 oder 13, **dadurch gekennzeichnet, dass** der Server (10, 111) ein Mobiltelefon umfasst.

15. Verfahren nach einem der Ansprüche 1 bis 14, **dadurch gekennzeichnet, dass** das Bereitstellen von Informationen und das Darstellen in Abhängigkeit von den Informationen nur durchgeführt wird, wenn die Anwendung eine fahrzeugfremde Anwendung ist.

16. Verfahren nach Anspruch 13, **dadurch gekennzeichnet, dass** das Bereitstellen von Informationen und das Darstellen in Abhängigkeit von den Informationen nur durchgeführt wird, wenn die Anwendung eine fahrzeugfremde Anwendung ist, und dass festgestellt wird, dass eine Anwendung eine fahrzeugfremde Anwendung ist, wenn der Server (111) ein fahrzeugfremder Server ist.

17. Verfahren nach einem der Ansprüche 1 bis 16, **dadurch gekennzeichnet, dass** das Darstellen in Abhängigkeit von einem Typ des Fahrzeugs (110) erfolgt.

18. Verfahren nach einem der Ansprüche 1 bis 17, **dadurch gekennzeichnet, dass** das Darstellen der Anwendung auf dem Bildschirm eine Darstellung eines Warnhinweises in Abhängigkeit von den Informationen hinsichtlich des Ablenkungsgrades umfasst.

19. Verfahren nach einem der Ansprüche 1 bis 18, **dadurch gekennzeichnet, dass** das Darstellen der Anwendung in Abhängigkeit von den Informationen hinsichtlich des Ablenkungsgrades eine Abfrage an einen Benutzer, ob die Anwendung dargestellt werden soll, in Abhängigkeit von dem Ablenkungsgrad umfasst.

20. Vorrichtung, umfassend:
einen Bildschirm (14),
wobei die Vorrichtung (13) zum Empfangen von Daten zur Darstellung einer Anwendung auf dem Bildschirm (14), zum Empfangen von Informationen hinsichtlich eines Ablenkungsgrades und zum Darstellen der Anwendung auf dem Bildschirm (14) in Abhängigkeit von den Informationen hinsichtlich eines Ablenkungsgrades und einer Fahrsituation eingerichtet ist,
**dadurch gekennzeichnet, dass** die Informationen hinsichtlich des Ablenkungsgrades Informationen hinsichtlich eines Ablenkungsgrades der Anwendung umfassen, und
dass das Darstellen in Abhängigkeit von einer Position des Bildschirms (14) in dem Fahrzeug (110) erfolgt.

21. Vorrichtung nach Anspruch 20, **dadurch gekennzeichnet, dass** die Vorrichtung zur Durchführung des Verfahrens nach einem der Ansprüche 1 bis 19 ausgestaltet ist.

## Claims

1. Method for displaying an application in a vehicle (110), comprising:
providing data for displaying an application on a screen (14),
providing information relating to a degree of deflection, and
displaying the application on the screen (14) on the basis of the information relating to the degree of deflection and a driving situation of the vehicle (110) ,
**characterized in that** the provision of information relating to a degree of deflection comprises providing information relating to a degree of deflection of the application, and
**in that** display is carried out on the basis of a position of the screen (14) in the vehicle (110).

2. Method according to Claim 1, **characterized in that** the display of the application on the basis of the information relating to the degree of deflection and the driving situation of the vehicle (110) comprises deciding whether the application is displayed on the screen (14).

3. Method according to Claim 1 or 2, **characterized in that** the application comprises a non-vehicle application.

4. Method according to Claim 3, **characterized in that** the information relating to the degree of deflection comprises information relating to user interface elements (15-18) of the application.

5. Method according to Claim 4, **characterized in that** the method also comprises calculating a size of the user interface elements (15-18) on the basis of a size and/or resolution of the screen (14).

6. Method according to Claim 4 or 5, **characterized in that** the display is carried out on the basis of a size of the user interface elements, a number of the user interface elements and/or a configuration of the user interface elements.

7. Method according to one of Claims 1 to 6, **characterized in that** the provision of information relating to a degree of deflection comprises determining information relating to a degree of deflection of a driver of the vehicle on the basis of sensor information.

8. Method according to Claim 7, **characterized in that** the sensor information comprises at least one item of information from the group consisting of image data from a camera and information from operating elements of the vehicle.

9. Method according to Claim 7 or 8, **characterized in that** the degree of deflection of the driver is captured only when the driver interacts with an operating device comprising the screen (14).

10. Method according to one of Claims 1 to 9, **characterized in that** the information relating to a degree of deflection comprises a certificate of the application or an item of information derived from the certificate.

11. Method according to one of Claims 1 to 10, **characterized in that** the driving situation is selected from the group comprising a moving vehicle, a stationary vehicle, a vehicle with the ignition switched off and a vehicle with the ignition switched on.

12. Method according to one of Claims 1 to 11, **characterized in that** the provision of the information relating to the degree of deflection comprises transmitting the information from a server (10, 111) to a client comprising the screen (14).

13. Method according to one of Claims 1 to 12, **characterized in that** the provision of data for displaying an application is effected by transmitting the data from a server (10, 111) to a client (13) comprising the screen (14).

14. Method according to either of Claims 12 and 13, **characterized in that** the server (10, 111) comprises a mobile telephone.

15. Method according to one of Claims 1 to 14, **characterized in that** the provision of information and the display on the basis of the information are carried out only if the application is a non-vehicle application.

16. Method according to Claim 13, **characterized in that** the provision of information and the display on the basis of the information are carried out only if the application is a non-vehicle application, and **in that** it is determined that an application is a non-vehicle application if the server (111) is a non-vehicle server.

17. Method according to one of Claims 1 to 16, **characterized in that** the display is carried out on the basis of a type of the vehicle (110).

18. Method according to one of Claims 1 to 17, **characterized in that** the display of the application on the screen comprises displaying a warning message on the basis of the information relating to the degree of deflection.

19. Method according to one of Claims 1 to 18, **characterized in that** the display of the application on the basis of the information relating to the degree of deflection comprises a query to a user, with regard to whether the application is intended to be displayed, on the basis of the degree of deflection.

20. Apparatus comprising:
a screen (14),
the apparatus (13) being set up to receive data for displaying an application on the screen (14), to receive information relating to a degree of deflection and to display the application on the screen (14) on the basis of the information relating to a degree of deflection and a driving situation,
**characterized in that** the information relating to the degree of deflection comprises information relating to a degree of deflection of the application, and
**in that** the display is carried out on the basis of a position of the screen (14) in the vehicle (110).

21. Apparatus according to Claim 20, **characterized in that** the apparatus is configured to carry out the method according to one of Claims 1 to 19.

## Revendications

1. Procédé servant à représenter une application dans un véhicule (110), consistant à :
fournir des données permettant la représentation d'une application sur un écran (14),
fournir des informations concernant un degré de distraction et
représenter l'application sur l'écran (14) en fonction des informations concernant le degré de distraction et d'une situation de conduite du véhicule (110),
**caractérisé en ce que** la fourniture d'informations concernant un degré de distraction consiste à fournir des informations concernant un degré de distraction de l'application, et
**en ce que** la représentation est effectuée en fonction d'une position de l'écran (14) dans le véhicule (110).

2. Procédé selon la revendication 1, **caractérisé en ce que** la représentation de l'application en fonction des informations concernant le degré de distraction et de la situation de conduite du véhicule (110) consiste à décider si l'application est représentée sur l'écran (14).

3. Procédé selon la revendication 1 ou 2, **caractérisé en ce que** l'application comprend une application étrangère au véhicule.

4. Procédé selon la revendication 3, **caractérisé en ce que** les informations concernant le degré de distraction comprennent des informations concernant des éléments d'interface utilisateur (15-18) de l'application.

5. Procédé selon la revendication 4, **caractérisé en ce que** le procédé consiste en outre à calculer une taille des éléments d'interface utilisateur (15-18) en fonction d'une taille et/ou d'une résolution de l'écran (14) .

6. Procédé selon la revendication 4 ou 5, **caractérisé en ce que** la représentation est effectuée en fonction d'une taille des éléments d'interface utilisateur, d'un nombre des éléments d'interface utilisateur et/ou d'une configuration des éléments d'interface utilisateur.

7. Procédé selon l'une quelconque des revendications 1 à 6, **caractérisé en ce que** la fourniture d'informations concernant un degré de distraction consiste à déterminer sur la base d'informations de capteurs des informations concernant un degré de distraction d'un conducteur du véhicule.

8. Procédé selon la revendication 7, **caractérisé en ce que** les informations de capteurs comprennent au moins une information appartenant au groupe constitué de données d'images d'une caméra et d'informations d'éléments de commande du véhicule.

9. Procédé selon la revendication 7 ou 8, **caractérisé en ce que** la détection du degré de distraction du conducteur n'est effectuée que lors d'une interaction entre le conducteur et un dispositif de commande comprenant l'écran (14).

10. Procédé selon l'une quelconque des revendications 1 à 9, **caractérisé en ce que** l'information concernant le degré de distraction comprend un certificat de l'application ou une information dérivée du certificat.

11. Procédé selon l'une quelconque des revendications 1 à 10, **caractérisé en ce que** la situation de conduite est sélectionnée dans le groupe comprenant un véhicule en déplacement, un véhicule à l'arrêt, un véhicule dont l'allumage est désactivé et un véhicule dont l'allumage est activé.

12. Procédé selon l'une quelconque des revendications 1 à 11, **caractérisé en ce que** la fourniture des informations concernant le degré de distraction consiste à envoyer les informations d'un serveur (10, 111) à un client comprenant l'écran (14).

13. Procédé selon l'une quelconque des revendications 1 à 12, **caractérisé en ce que** la fourniture de données destinées à représenter une application est effectuée par transmission des données d'un serveur (10, 111) à un client (13) comprenant l'écran (14).

14. Procédé selon l'une quelconque des revendications 12 ou 13, **caractérisé en ce que** le serveur (10, 111) est un téléphone mobile.

15. Procédé selon l'une quelconque des revendications 1 à 14, **caractérisé en ce que** la fourniture d'informations et la représentation en fonction des informations ne sont effectuées que lorsque l'application est une application étrangère au véhicule.

16. Procédé selon la revendication 13, **caractérisé en ce que** la fourniture d'informations et la représentation en fonction des informations ne sont effectuées que lorsque l'application est une application étrangère au véhicule et **en ce qu'**il est établi qu'une application est une application étrangère au véhicule lorsque le serveur (111) est un serveur étranger au véhicule.

17. Procédé selon l'une quelconque des revendications 1 à 16, **caractérisé en ce que** la représentation est effectuée en fonction du type du véhicule (110).

18. Procédé selon l'une quelconque des revendications 1 à 17, **caractérisé en ce que** la représentation de l'application sur l'écran comprend la représentation d'un avertissement en fonction des informations concernant le degré de distraction.

19. Procédé selon l'une quelconque des revendications 1 à 18, **caractérisé en ce que** la représentation de l'application en fonction des informations concernant le degré de distraction consiste à demander à un utilisateur si l'application doit être représentée en fonction du degré de distraction.

20. Dispositif, comprenant :
un écran (14),
dans lequel le dispositif (13) est conçu pour recevoir des données permettant la représentation d'une application sur l'écran (14), pour recevoir des informations concernant un degré de distraction et pour représenter l'application sur l'écran (14) en fonction des informations concernant le degré de distraction et d'une situation de conduite,
**caractérisé en ce que** les informations concernant le degré de distraction comprennent des informations concernant un degré de distraction de l'application, et **en ce que** la représentation est effectuée en fonction d'une position de l'écran (14) dans le véhicule (110).

21. Dispositif selon la revendication 20, **caractérisé en ce que** le dispositif est configuré pour mettre en oeuvre le procédé selon l'une quelconque des revendications 1 à 19.
